(19) Europäisches Patentamt
European Patent Office
Office européen des brevets

(11) Veröffentlichungsnummer: **0 315 638 B1**

# (12) EUROPÄISCHE PATENTSCHRIFT

(45) Veröffentlichungstag der Patentschrift: **09.01.91**

(51) Int. Cl.$^5$: **A 61 K 31/63**

(21) Anmeldenummer: **87905203.3**

(22) Anmeldetag: **30.07.87**

(86) Internationale Anmeldenummer:
**PCT/EP87/00417**

(87) Internationale Veröffentlichungsnummer:
**WO 88/00828 11.02.88 Gazette 88/04**

## (54) Verwendung von Sulfonamidderivaten zur Herstellung eines Antivirusmittels.

(30) Priorität: **02.08.86 DE 3626309**

(43) Veröffentlichungstag der Anmeldung:
**17.05.89 Patentblatt 89/20**

(45) Bekanntmachung des Hinweises auf die Patenterteilung:
**09.01.91 Patentblatt 91/02**

(84) Benannte Vertragsstaaten:
**AT BE CH DE FR GB LI LU NL SE**

(56) Entgegenhaltungen:
**DE-A-2 433 723**

(73) Patentinhaber: **Chemische Fabrik Stockhausen GmbH**
**Bäkerpfad 25**
**D-4150 Krefeld (DE)**

(72) Erfinder: **Komp, Bernd, Dr.**
**Kortenbacherweg 48a**
**D-6453 Seligenstadt (DE)**
Erfinder: **Kresken, Joachim, Dr.**
**Elsternweg 8**
**D-4150 Krefeld-Forstwald (DE)**
Erfinder: **Stockhausen, Dolf, Dr.**
**Brahms-Strasse 104**
**D-4150 Krefeld (DE)**

(74) Vertreter: **Klöpsch, Gerald, Dr.-Ing.**
**Patentanwälte Klöpsch & Flaccus**
**An Gross St. Martin 6**
**D-5000 Köln 1 (DE)**

EP 0 315 638 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Verwendung von Sulfonamidderivaten der 4,4'-Diaminostilbensulfosäure-2,2' und/oder der 4,4-Diaminodiphenylethandisulfosäure-2,2' zur Herstellung eines Mittels zur Bekämpfung und Verhütung von Viruserkrankungen, insbesondere von durch eine Umhüllung aufweisende Viren hervorgerufenen Erkrankungen.

Solche Sulfonamidderivate der allgemeinen Formel

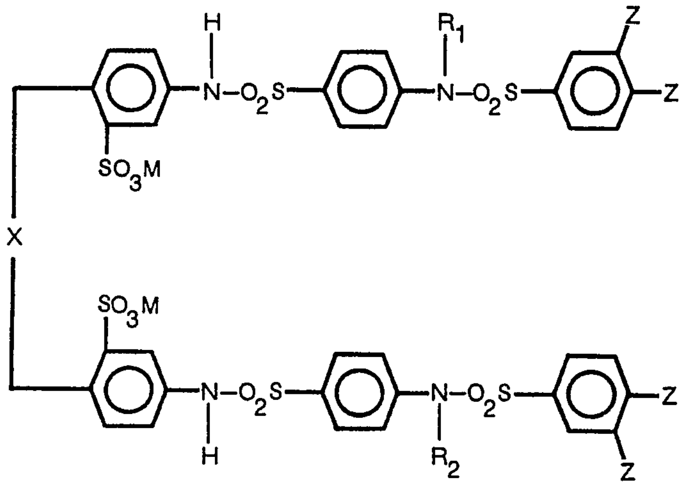

worin X für die Gruppe —CH=CH— oder $—CH_2—CH_2—$, Z für ein Chlor- oder Bromatom, M für ein nichttoxisches Kation, bevorzugt Alkali oder Ammonium stehen und $R_1$ und $R_2$, die gleich oder ungleich sind, ein Wasserstoffatom oder den 3,4-Dihalogenbenzolsulfonyl-Rest bedeuten und ihre Herstellung sind bereits in der DE—PS 2433723 beschrieben.

Diese Produkt absorbieren Strahlen (insbesondere UV-Strahlen), wirken eiweißkoagulierend und werden als Wirkkomponente in Hautschutzmitteln eingesetzt.

Es hat sich nun überraschenderweise gezeigt, daß die vorstehend beschriebenen Sulfonamidderivate eine hohe virucide und antivirale Wirkung, insbesondere gegenüber mit einer Umhüllung versehenen Viren entfalten, ohne dabei zellschädigende Nebenwirkungen zu entwickeln.

Ein typisches Beispiel solcher "umhüllter" Viren sind Herpes-Viren, insbesondere Herpes simplex, aber auch das Varicella-Zoster-Virus.

Durch Herpes simplex-Viren erzeugte Erkrankungen wie Herpes labialis und Herpes genitalis können mit den heute verfügbaren Therapiemethoden nur sehr unbefriedigend behandelt werden [Wassilew, Sawko W., Möglichkeiten und Grenzen der Chemotherapie bei Erkrankungen durch das Herpes-simplex- und das Varicella-Zoster-Virus, Der Hautarzt, *34*, 1 bis 5 (1983); Wassilew, Sawko W., Virustatika in der Dermatologie, Der Hautarzt, *37*, 259—262 (1986); Ring, J., Fröhlich, H. H., Wirkstoffe in der dermatologischen Therapie, 140 bis 143, Springer Verlag (1985)].

Die meisten der bisher eingesetzten Produkte erweisen sich in klinischen Studien entweder als unwirksam oder aber sind mit unakzeptablen Nebenwirkungen verbunden. Hierzu zählen in erster Linie toxische Einflüsse auf die vom Virus befallene Wirtszelle, aber auch z.B. die Auslösung von Allergien (siehe Wassilew, Sawko a.a.O.).

Aufgabe der Erfindung ist also die Bereitstellung eines wirksamen Antivirus-Mittels, das insbesondere zur Bekämpfung und Verhütung von durch umhüllte Viren, wie Herpes-simplex-Viren der Typen 1 und 2 hervorgerufene Erkrankungen geeignet ist, und das keine oder möglichst geringe Nebenwirkungen hat.

Diese Aufgabe wird durch die Verwendung der eingangs genannten speziellen Sulfonamidderivate als Wirkstoffe zur Herstellung eines Mittels zur Virusbekämpfung, insbesondere von umhüllten Viren, wie Herpes-Viren, gelöst.

Die erfindungsgemäß zu verwendenden Produkte komme in einer pharmazeutischen Zubereitung in 0,1 bis 20%iger Konzentration (Gew.-%), gegebenenfalls zusammen mit pharmazeutisch akzeptablen Trägern, Verdünnungsmitteln und/oder Hilfsstoffen zur Anwendung. Bei den Träger- bzw. Hilfsstoffen handelt es sich um übliche aus der Galenik bekannte Stoffe; als Träger bzw. Verdünnungsmittel können Wasser, niedere ein- und mehrwertige Alkohole wie Glycerin sowie Paraffinöl oder Vaseline eingesetzt werden. Die erfindungsgemäß zu verwendenden Produkte können somit in allen üblichen pharmazeutischen Darreichungsformen angewendet werden. Die topische Anwendung, z.B. als Creme, Salbe, Paste, Gel, oder Lösung, ist bevorzugt.

Die genannten Sulfonamidderivate können aber nicht nur zur Therapie, sondern auch in Kosmetikartikeln, wie z.B. Lippenstift, Intimhydrieneprodukten, oder speziellen Hautschutzprodukten, zur Infektionsprophylaxe und Vorbeugung gegen weitere Ausbreitung von Viren eingesetzt werden. Auch

können sie als Zusätze in Hautreinigungsmitteln, wie Seifen, oder in Kombination mit Desinfektionsmitteln angewendet werden.

An einschichtigen Zellkulturen von Nierenzellen (Affe), die mit Herpes-simplex-Viren beimpft worden sind, zeigen die erfindungsgemäßen Verbindungen in Testkonzentrationen von 25 bis 100 µg/ml eine bis zu 99,999%ige Hemmung des Virus-Titers. Ein toxischer Einfluß auf die Wirtszelle tritt erst ab einer Konzentration von etwa 250 µg/ml auf.

Im Gegensatz zu den erfindungsgemäß zu verwendenden Produkten zeigen die meisten der bisher bekannten antiviralen Mitteln entweder eine unzureichende Hemmwirkung oder aber bei ausreichender Wirkung schon eine deutlich toxische Wirkung auf die Wirtszelle, gemessen an deren Proteinsyntheseleistung.

Besonders vorteilhaft erweist sich bei den erfindungsgemäß zu verwendenden Produkten, daß sie auch eine extrazelluläre virucide Wirkung haben, wodurch auch ein desinfizierender und — wie bereits weiter oben erwähnt — Einsatz in der Prophylaxe möglich ist,

Die extrazelluläre virucide Wirkung kommt über eine elektronenmikroskopisch nachvollziehbare Schädigung der Hülle der Viren zustande, wodurch diese ihre Infektiosität verlieren.

Zur quantitativen Erfassung der viruciden Wirkung werden die Herpes-simplex-Viren zunächst mit den erfindungsgemäßen Verbindungen inkubiert. Im Anschluß daran wird die Restinfektiosität auf die Wirkszellen bestimmt.

Sowohl die antivirale als auch die virucide Wirksamkeit der erfindungsgemäß zu verwendenden Produkte führen, bestimmt nach der Endpunkttitrationstechnik, zu einer Verringerung des ursprünglich vorhandenen Virustiters durch die wirksame Verbindung um bis zu 99,999% für Herpes-simplex-Viren.

Die Wirksamkeit der erfindungsgemäß zu verwendenden Produkte erweist sich auch in einem in-vivo-Modell an weißen Mäusen. Hierbei werden die Tiere entweder vor oder nach einer mit Herpes-simplex-Viren durchgeführten Infizierung des Rückens mit den zu prüfenden Verbindungen, die z.B. in einer Lösung oder Creme vorliegen, behandelt.

## Experimentelle Untersuchungen

In Vitro-Versuche

*Bestimung der antiviralen und viruciden Wirkung*

Material und Methoden

Für die nachfolgenden Untersuchungen wurden Lösungen der erfindungsgemäß zu verwendenden Wirkstoffe in Wasser oder in DMSO/Wasser-Mischungen hergestellt und diese mit Nährmedium oder isotonischem Phosphatpuffer auf 4 verschiedene Testkonzentrationen (100, 75, 50 und 25 µg/ml) verdünnt.

a) Antivirale Wirksamkeit

Ausgehend von einer Suspension von Herpes-simplex-Viren (Typ 1 oder 2) in Nährmedium ($TCD_{50}$/ml: $10^6$) wurden sieben, jeweils um den Faktor 10 verdünnte Lösungen hergestellt ($TCD_{50}$/ml: $10^5$—$10^{-1}$). Mit jeweils 0,1 ml dieser Vedünnungen wurden auf Microtiterplatten Monoschichten von ZERO-Zellen infiziert und 90 Minuten bei 37°C bebrütet. Anschließend wurden jeweils 0,1 ml der 4 Konzentrationen der Testverbindungen zu den verschiedenen Viruskonzentrationen hinzugegeben.

Die Platten wurden in einem Feuchtinkubator bei 37°C 5 Tage bebrütet. Eine Cytotoxizitäts-, Zell- und Viruskontrolle wurden für jedes Konzentrationsverhältnis parallel durchgeführt. Der cytopathogene Effekt auf die Monoschichten wurde täglich beobachtet.

b) Virucide Wirksamkeit

Gleiche Volumina der vier Konzentrationen an den zu prüfenden Verbindungen wurden mit einer Suspension von Herpes-simplex-Viren (Typ 1 oder 2, $TCD_{50}$/ml: $10^5$) 1 Stunde bei 37°C inkubiert. Danach wurden von den 4 Ausgangssuspensionen 7 jeweils um den Faktor 10 verdünnte Lösungen hergestellt. Jeweils 0,2 ml dieser Lösungen wurden dann zur Bestimmung des noch vorhandenen Virustiters in mit Monoschichten von VERO-Zellen beschickte Microtiterplatten gegeben. Die Platten wurden in einem Feuchtinkubator 5 Tage bei 37°C bebrütet. Ferner wurde ein Blindversuch (ohne Testverbindung) durchgeführt. Der cytopathogene Effekt wurde täglich beobachtet.

Versuchsergebnisse:

Die antivirale und virucide Wirkung der untersuchten Verbindungen werden ausgedrückt als Reduktionsfaktor, um den der anfängliche Virustiter verringert worden ist.

in der folgenden Tabelle 1 sind bei gleichen Wirkstoffkonzentrationen antivirale und virucide Wirkung auf Herpes-simplex-Viren Typ 1 oder 2 gegenübergestellt.

TABELLE 1

Ergebnisse der in vitro-Tests

| Substanz/ Konzentration | | antivirale Wirkung (ausgedrückt als Reduktionsfaktor) | virucide Wirkung (ausgedrückt als Reduktionsfaktor) |
|---|---|---|---|
| Wirkstoff gemäß Erfindung | 100 µg/ml | $10^4$—$10^5$ | $10^4$—$10^5$ |
| dto. | 75 µg/ml | $10^3$—$10^4$ | $10^3$—$10^4$ |
| dto. | 50 µg/ml | $10^2$—$10^3$ | $10^2$—$10^3$ |
| dto. | 25 µg/ml | 10 —$10^2$ | 10 —$10^2$ |

Die angegebenen Intervalle ergeben sich aufgrund unterschiedlicher Probenaufbereitung und den damit verbundenen Abweichung in der Löslichkeit.

Die Substanzen bewirken in den getesteten Konzentrationen keine morphologischen Veränderungen der Zellen.

In vivo-Untersuchungen

Material und Methoden

Mäuse vom Typ Mus Musculus Mutand Gentyp HR/HR (Alter 3 Wochen) wurden in Gruppen zu je 8—12 Tieren eingeteilt und mit je 100 µl einer Suspension von Herpes-simplex-Viren Typ 1 oder 2 ($10^6$ $TCD_{50}$/ml) infiziert.

Die Behandlung der Tiere erfolgte nach verschiedenen Schemata:

Behandlungsschema a: Behandlung 48, 24 und 1,5 Stunden vor der Infektion.

Behandlungsschema b: Behandlung 1,5 Stunden nach der Infektion und dann 2 × täglich über 10 Tage.

Die Versuchsergebnisse sind in der folgenden Tabelle zusammengefaßt:

TABELLE 2

Ergebnisse der in vivo-Versuche

| Substanz/ Konzentration | | Vehikel | Behandlungs-schema | Klinische Ergebnisse (% Versuchstiere mit normalen Herpes-Läsionen*) |
|---|---|---|---|---|
| Wirkstoff gemäß Erfindung | 2% | DMSO/Wasser (30:70) | a | 0 |
| dto. | 2% | dto. | b | 75 |
| dto. | 5% | dto. | a | 5 |
| dto. | 5% | dto. | b | 80 |
| dto. | 1% | Wasser | a | 0 |
| dto. | 10% | O/W-Creme | a | 16 |
| dto. | 10% | dto. | b | 88 |
| Kontrolle ohne Wirkstoff | — | DMSO/Wasser (30:70) | a | 100 |
| dto. | — | dto. | b | 100 |

* Innerhalb von 3 bis 4 Tagen nach der Infektion treten kleine weiße Knötchen auf, die sich nach weiteren 6 bis 7 Tagen zu charakteristischen Herpes-Läsionen entwickeln.

**Patentansprüche**

1. Verwendung von Sulfonamidderivaten der allgemeinen Formel:

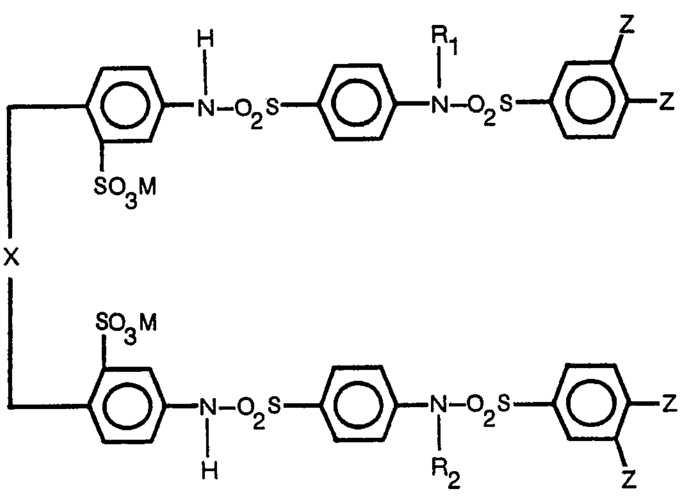

worin X für die Gruppe —CH=CH— oder —$CH_2CH_2$—, Z für ein Chlor- oder Bromatom, M für ein nichttoxisches Kation, bevorzugt Alkali oder Ammonium stehen und $R_1$ und $R_2$ gleich oder ungleich sind, ein Wasserstoffatom oder den 3,4-Dihalogenbenzolsulfonylrest, bevorzugt den 3,4-Dichlorbenzolsulfonylrest bedeuten, als Wirkstoff zur Herstellung eines Arzneimittels zur Behandlung und Vorbeugung von durch Viren, insbesondere von umhüllten Viren, erzeugte Erkrankungen, gegebenenfalls zusammen mit pharmazeutisch unbedenklichen Trägern, und/oder Hilfsstoffen.

2. Verwendung nach Anspruch 1, wobei der Wirkstoffgehalt von 0,1 bis 20 Gew.-% beträgt.

## Revendications

1. Utilisation de dérivés de sulfonamides de la formule générale suivante:

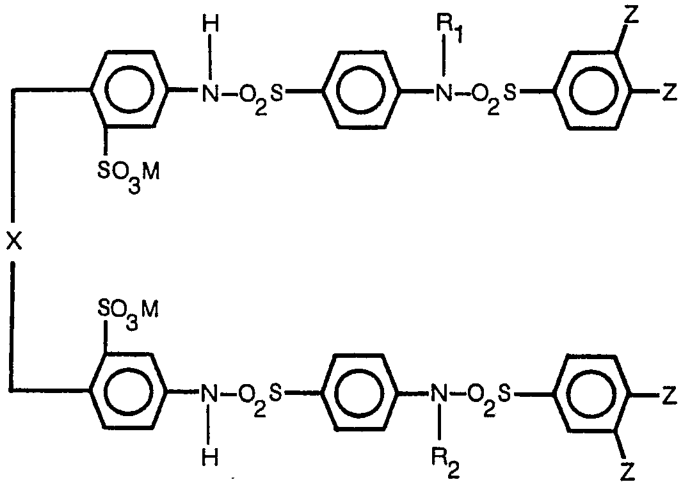

dans laquelle X représente le radical —CH=CH— ou —$CH_2$—$CH_2$—, Z représente un atome de chlore ou de brome, M représente un cation atoxique, de préférence, de métal alcalin ou d'ammonium et $R_1$ et $R_2$ sont identiques ou différents et représentent chacun un atome d'hydrogène ou la radical 3,4-dihalogénobenzènesulfonyle, de préférence le radical 3,4-dihalogènobenzènesulfonyle, à titre de substance active pour la préparation d'un médicament destiné au traitement et à la prévention de maladies causées par des virus, plus particulièrement, des virus à enveloppe, éventuellement conjointement avec des véhicules, excipients et/ou des adjuvants pharmaceutiquement compatibles.

2. Utilisation suivant la revendication 1, caractérisée en ce que la teneur en substance active varie de 0,1 à 20% en poids.

## Claims

1. Use of sulphonamide derivatives of the general formula:

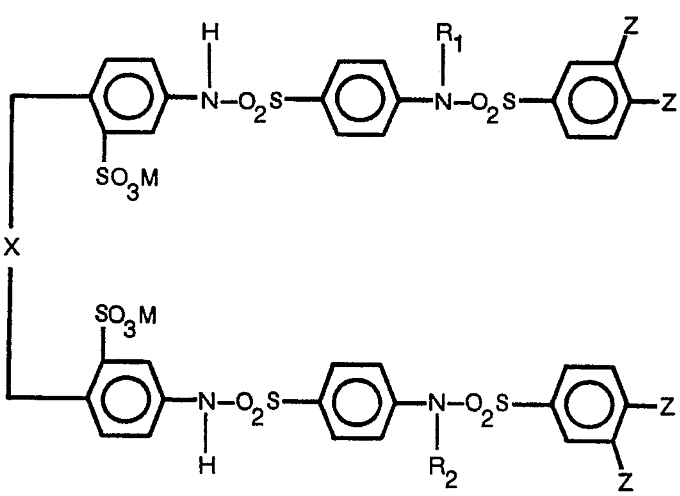

wherein X represents the group —CH=CH— or —$CH_2$—$CH_2$—, Z represents an atom of chlorine or bromine, M represents a non-toxic cation, preferably alkali or ammonium, and $R_1$ and $R_2$ which are the same or different represent a hydrogen atom, or the 3,4-dihalogenobenzene sulphonyl group, preferably the 3,4-dichlorobenzene sulphonyl group, as active substance for the production of a medicine for the treatment and prevention of diseases caused by viruses, particularly by enclosed viruses, optionally together with pharmaceutically acceptable carriers and/or auxiliaries.

2. The use according to claim 1 whereby the active substance content amounts to 0.1 to 20%-wt.